# EUROPÄISCHE PATENTSCHRIFT

(11) **EP 2 243 434 B1**
(45) Veröffentlichungstag und Bekanntmachung des Hinweises auf die Patenterteilung: **04.01.2012**
(21) Anmeldenummer: 10151051.9
(22) Anmeldetag: 19.01.2010
(51) Int. Cl.: A61B 17/128

(54) **Chirurgisches Instrument zum Anlegen von Ligaturklammern**
Surgical instrument for the placement of ligating clips
Dispositif chirurgical pour la mise en place de clips de ligature

(30) Priorität: 24.04.2009 DE 102009018818
(43) Veröffentlichungstag der Anmeldung: 27.10.2010
(73) Patentinhaber: AESCULAP AG, 78532 Tuttlingen (DE)
(72) Erfinder: Disch, Alexander, 79104 Freiburg (DE); Mayenberger, Rupert, 78239, Rielasingen (DE)
(74) Vertreter: Hoeger, Stellrecht & Partner Patentanwälte

(56) Entgegenhaltungen:
- EP-A2- 0 538 074
- WO-A1-03/086207
- US-B1- 6 440 144

## Beschreibung

Die Erfindung betrifft ein chirurgisches Instrument zum Anlegen von C-förmigen, zwei über einen Brückenabschnitt miteinander verbundene Schenkel aufweisenden Ligaturklammern mit zwei in einer Offenstellung eine offene Ligaturklammer zwischen sich aufnehmenden und durch gegenseitige Annäherung in eine Schließstellung bewegbaren und dabei die Ligaturklammer durch Annäherung ihrer Schenkel schließenden Klemmbacken, mit zwei elastisch verformbaren Armen, die an ihrem distalen Ende je einen Klemmbacken tragen, und mit einem in Längsrichtung der Arme relativ zu diesen verschiebbaren Schieber, der an Anlageflächen der Arme anliegt und bei einer Verschiebung des Schiebers relativ zu den Armen durch diese Anlage an den Anlageflächen die Arme in einer Verschwenkebene elastisch aus einer Anfangsstellung in eine Endstellung gegen einander verschwenkt, wobei die beiden Arme zumindest über einen Teil ihrer Länge und zumindest über einen Teil ihrer Breite in parallel zu der Verschwenkebene verlaufenden und gegen einander versetzten Ebenen angeordnet sind und sich zumindest teilweise überlappen, dadurch gekennzeichnet, dass im Überlappungsbereich den innen liegenden Seitenflächen der Arme kein Teil des Schiebers gegenüber liegt.

Derartige chirurgische Instrumente sind beispielsweise beschrieben in der US 3,777,538 A, der DE 30 21 099 A1, der DE 696 36 965 T2 oder der DE 696 34 391 T2. Die als Biegearme ausgebildeten Arme sind dabei einstückig miteinander verbunden und werden durch einen Schieber, der die Arme überfängt und der an nockenbahnähnlichen Anlageflächen entlang gleitet, wenn der Schieber und die Arme in Längsrichtung relativ zueinander verschoben werden, aus der Offenstellung in die Schließstellung verschwenkt, dabei bewegen sich die Arme in allen Fällen in derselben Ebene, das heißt bei einer vollständigen Annäherung stoßen die Arme gegen einander.

Chirurgische Instrumente dieser Art werden vorwiegend für minimalinvasive Eingriffe eingesetzt, bei denen die Instrumente durch in der Regel rohrförmige Trokare durch kleine Körperöffnungen in den Körper eingeführt werden, und dementsprechend sind die Platzverhältnisse sehr beengt. Die nebeneinander liegenden, gegeneinander verschwenkbaren Arme müssen sich innerhalb des sehr engen Querschnitts eines schaftförmigen Instrumententeils bewegen, und dies führt dazu, dass der Schwenkwinkel der Arme eingeschränkt ist. Im ausgeschwenkten Zustand dürfen die Arme nicht über die Kontur eines Schaftes des Instrumentes hervorstehen. Daher können die Arme, die in der eingeschwenkten Endstellung mit ihren Innenflächen aneinander anliegen, nur über einen geringen Winkelbereich verschwenkt werden, und dies ist unter Umständen für die Aufnahme von Ligaturklammern nicht ausreichend. Diese Ligaturklammern sollen an Gefäße und andere Körperteile angesetzt werden und sollen in der Offenstellung einen möglichst großen Abstand zwischen den nebeneinander liegenden Schenkeln aufweisen, so dass sie von den Klemmbacken des Instrumentes nur aufgenommen werden können, wenn diese einen genügend großen Abstand voneinander haben. In der WO03/086207 A ist ein weiteres gattungsgemäßes Instrument beschrieben, bei dem die beiden nebeneinander liegenden Arme jeweils zum anderen Arm hin gerichtete Querstege aufweisen, die in Ausnehmungen am gegenüberliegenden Arm eingreifen, wenn die Arme geschlossen sind. Dadurch wird eine bessere Führung der beiden Arme relativ zueinander erreicht, es ergibt sich aber keine Vergrößerung des Schwenkwinkels der Arme bei eingeschränktem Querschnitt des Instrumentes.

In der US 6,440,144 B1 ist ein Instrument beschrieben gemäß den Oberbegriff des Anspruchs 1, bei dem die Arme in parallel zueinander versetzten Ebenen verschwenkt werden, so dass sie sich im Schließzustand teilweise überlappen. Dies führt zu einer Vergrößerung des Verschiebeweges der Arme auch bei beengten Querschnittsverhältnissen des Instrumentes, allerdings wird diese Vergrößerung der Verschiebebewegung dadurch begrenzt, dass ein über die Arme geschobener Schieber, durch den die Schließbewegung der Arme erzeugt wird, einen Teil des Querschnittes einnimmt, der dadurch für die Schwenkbewegung der Arme nicht mehr zur Verfügung steht.

Es ist Aufgabe der Erfindung ein gattungsgemäßes chirurgisches Instrument so auszubilden, dass trotz der sehr beengten Platzverhältnisse eine genügend große Verschwenkbewegung der Arme möglich ist, um die Klemmbacken auch für offene Ligaturklammern ausreichend weit voneinander entfernen zu können.

Diese Aufgabe wird bei einem chirurgischen Instrument der eingangs beschriebenen Art erfindungsgemäß dadurch gelöst, dass im Überlappungsbereich den innenliegenden Seitenflächen der Arme kein Teil des Schiebers gegenüberliegt, so dass die Arme bei dem Verschwenken in die Endstellung den gesamten ihnen zur Verfügung stehenden Querschnitt des Instrumentes ausnützen können, ohne dass sie gegen Teile des Schiebers stoßen. Der Schieber kann in diesem Bereich fensterartige Ausnehmungen aufweisen, es ist auch möglich, dass sich der Schieber an der Außenseite der Arme nur jeweils über die Höhe der Arme erstreckt und dadurch dem jeweils anderen Arm Raum für dessen Verschwenkbewegung in dessen Endstellung gibt.

Bei einer ersten bevorzugten Ausführungsform ist vorgesehen, dass einer der Arme im Überlappungsbereich vollständig in einer oberen Ebene angeordnet ist und der andere der Arme in einer unteren Ebene.

Bei einer abgewandelten Ausführungsform kann aber auch vorgesehen sein, dass einer der Arme im Überlappungsbereich zum anderen Arm hin offene Ausnehmung aufweist, in die der andere Arme zumindest teilweise eintaucht. Dann befinden sich die überlappenden Teile der beiden Arme jeweils in unterschiedlichen Ebenen und können sich gegenseitig nicht behindern, trotzdem können sich beide Arme über einen relativ großen Verschwenkbereich bewegen und gegebenenfalls in ihren Ebenen auch eine größere Breite aufweisen.

Es kann vorgesehen sein, dass sich die Arme bereits in ihrer Anfangsstellung, in der die Klemmbacken in der Offenstellung stehen, teilweise überlappen.

Weiterhin ist möglich, dass sich die Arme in ihrer Endstellung, in der die Klemmbacken in der Schließstellung stehen, zumindest bereichsweise über ihre gesamte Breite überlappen, also den gesamten zur Verfügung stehenden Raum ausfüllen können.

Die Überlappung kann über einen Teil der Länge der Arme erfolgen oder auch über die gesamte Länge der Arme.

Günstig ist es, wenn die Breite der Arme im Überlappungsbereich größer ist als deren Höhe. Das Biegemoment der Arme in der Verschwenkebene ist quadratisch von der Breite der Arme abhängig und nur linear von der Höhe der Arme, so dass eine Verbreiterung das Biegemoment stark erhöht. Durch die Anordnung der Arme in unterschiedlichen Ebenen und durch die Möglichkeit der Überlappung können die Arme breiter und gegebenenfalls bei sehr geringem Platzbedarf etwas weniger hoch ausgebildet werden, trotzdem ist dadurch nicht nur keine Reduzierung des Biegemomentes zu befürchten, sondern dieses kann eventuell sogar noch erhöht werden, obwohl der zur Verfügung Raum nicht größer ist als bei herkömmlichen Armen, die in einer Ebene gegeneinander verschwenkt werden.

Günstig ist es, wenn die außen liegenden Seitenflächen der Arme in der Anfangsstellung über die gesamte Länge der Arme mit Ausnahme des distalen Endbereiches innerhalb einer Kontur mit parallel zueinander verlaufender Begrenzung angeordnet sind. Die Arme sind also in der Anfangsstellung nicht nach außen ausgeschwenkt, sondern verlaufen mit ihren Außenflächen parallel zueinander, so dass sie innerhalb der Außenkontur eines Instrumentes aufgenommen werden können, welches sich zum distalen Ende hin nicht verbreitert.

Insbesondere kann vorgesehen sein, dass die Begrenzung durch die Seitenwände einer die Arme aufnehmenden Kammer des Schiebers gebildet wird. In diesem Falle begrenzt also der Schieber ein elastisches Aufbiegen der Arme, die dadurch in der Ruhestellung im Wesentlichen parallel zueinander verlaufen.

Bei einer bevorzugten Ausführungsform der Erfindung ist vorgesehen, dass der Schieber an seinem distalen Ende an nach außen abstehenden Anlageflächen der Arme anliegt, die sich unmittelbar proximal an die Klemmbacken anschließen. Damit erfolgt die Einleitung der Schließkräfte in die Arme in maximalem Abstand von der proximalen Verbindungsstelle der beiden Arme und unmittelbar neben den am distalen Ende der Arme angeordneten Klemmbacken. Günstig ist es, wenn die Anlageflächen der beiden Arme in unterschiedlichen Ebenen liegen, d.h. in unterschiedlicher Höhe in Bezug auf die Verschwenkebene der jeweiligen Arme.

Besonders vorteilhaft ist es, wenn die Anlageflächen jeweils in der Höhe der entsprechenden Arme angeordnet sind, also in der Schwenkebene dieser Arme. "In der Ebene" bedeutet dabei, dass die Anlageflächen nicht oberhalb oder unterhalb der Arme verlaufen, die Arme sind also gegenüber ihrer Verschwenkebene nicht abgekröpft oder abgebogen. Auch dies trägt dazu bei, dass die Baugröße des Instrumentes reduziert wird.

Es kann vorgesehen sein, dass die Arme zumindest an einem Teil ihrer Länge von dem Schieber allseits umgeben werden. Diese allseitige Umgebung führt zu einer Stabilisierung des Instrumentes und sichert die in unterschiedlichen Ebenen verschwenkbaren Arme gegen eine ungewollte Verformung, beispielsweise senkrecht zu den Verschwenkebenen.

Es ist vorteilhaft, wenn sich die Arme im unverformten Zustand über ihre gesamte Länge nicht überlappen. Dadurch wird die Herstellung der Arme erleichtert, eine Überlappung tritt erst ein, wenn diese gegeneinander verschwenkt werden, sei es durch den Einbau in das Instrument schon in der Anfangsstellung, sei es erst bei der Verschwenkung der Arme in die Endstellung.

Die Arme können von ihrer proximalen Verbindungsstelle zu ihrem distalen Ende ohne eine Überkreuzung nebeneinander her laufen, es ist aber auch möglich, dass sich die Arme zwischen ihrem proximalen Ende und ihrem distalen Ende überkreuzen.

Bei einer bevorzugten Ausführungsform ist vorgesehen, dass die Arme und der Schieber gemeinsam innerhalb eines rohrförmigen Schaftes mit einer kreisförmigen Kontur angeordnet sind, über die auch die Klemmbacken in ihrer Offenstellung nicht hervorstehen. Es ist dadurch möglich, das Instrument mit geöffneten Klemmbacken durch einen rohrförmigen Zugang in den Körper einzuführen.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Ansicht des Klemmbackenbereiches eines chirurgischen Instrumentes bei der Anlage einer Ligaturklammer an einem Gefäß;
- Figur 2:: eine perspektivische Ansicht des distalen Endes des chirurgischen Instrumentes der Figur 1 mit einem in das Instrument eingesetz- ten Magazin zur Aufnahme von zwischen die Klemmbacken ein- schiebbaren Ligaturklammer;
- Figur 3:: eine Ansicht ähnlich Figur 2 mit dem vom Instrument abgenom- menen Magazin;
- Figur 4:: eine perspektivische Draufsicht auf die Klemmbacken tragenden Arme des Instrumentes in einer Kammer eines Schiebers zum Schließen der Klemmbacken;
- Figur 5:: eine Ansicht ähnlich Figur 4 mit aus dem Schieber herausgenom- menen Armen;
- Figur 6:: eine perspektivische Ansicht der die Klemmbacken tragenden Arme im unverformten Zustand;
- Figur 7:: eine Ansicht ähnlich Figur 4 bei einem abgewandelten Ausfüh- rungsbeispiel eines Schiebers;
- Figur 8:: eine Schnittansicht längs Linie 8-8 in Figur 5 und
- Figur 9:: eine Ansicht ähnlich Figur 8 bei einer abgewandelten Quer- schnittsform der Arme.

Das in der Zeichnung dargestellte Instrument 1 umfasst einen rohrförmigen Schaft 2, von dem in der Zeichnung lediglich das distale Ende dargestellt ist.

An seinem in der Zeichnung nicht dargestellten proximalen Ende ist der Schaft 2 mit einem Handgriff verbunden, an dem Aktivierungsglieder angeordnet sind, durch die den Schaft durchsetzende Übertragungsmittel, beispielsweise Schub- und Zugstangen, bewegt werden können, deren Bewegung auf diese Weise zum distalen Ende des Schaftes 2 übertragen werden kann.

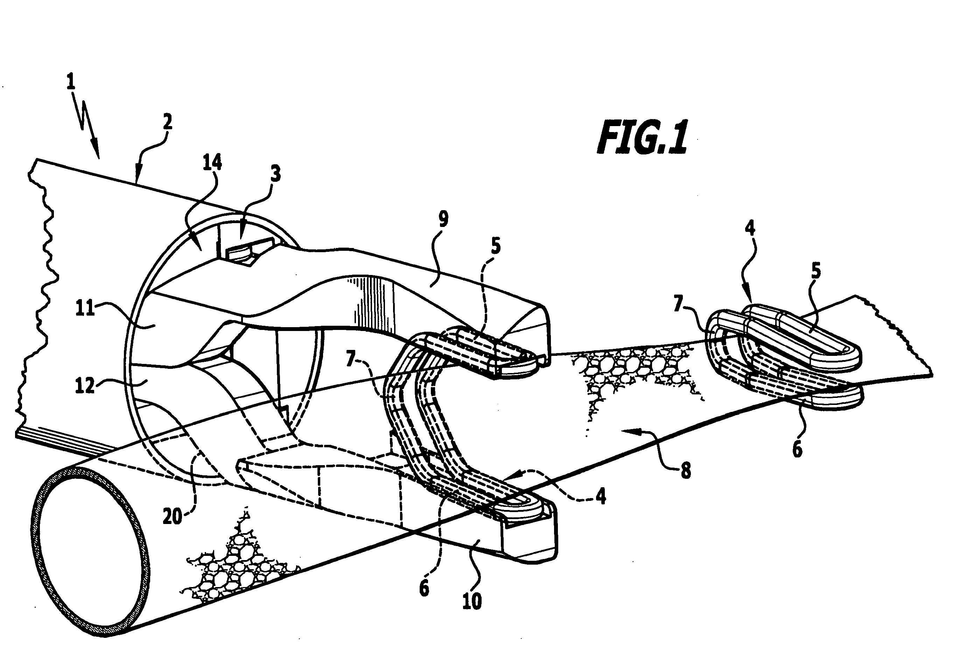

In den Schaft 2 ist an seinem distalen Ende ein Magazin 3 eingesetzt, das im Wesentlichen einen halbkreisförmigen Querschnitt aufweist und in dem eine Vielzahl von C-förmigen Ligaturklammern 4 angeordnet sind. Diese Ligaturklammern weisen zwei nebeneinander und im Abstand zu einander angeordnete Schenkel 5, 6 auf, die an ihrem proximalen Ende über einen Brückenabschnitt 7 miteinander verbunden sind. Sie bestehen aus einem dauerhaft verformbaren Material, insbesondere aus Metall, beispielsweise aus Titan oder einer Titanlegierung, die Schenkel 5, 6 können aus einer Offenstellung, in der sie einen Abstand zueinander aufweisen (linke Ligaturklammer in Figur 1) gegeneinander in eine Schließstellung gedrückt werden, in der die Schenkel einander angenähert sind und einen zwischen ihnen angeordneten Gefäßabschnitt 8 verschließen und im Klemmsitz an diesem Gefäßabschnitt 8 gehalten werden (rechte Ligaturklammer in Figur 1).

Zum Verformen der Ligaturklammern 4 aus der Offenstellung in die Schließstellung werden die Ligaturklammern 4 zwischen zwei im Abstand zueinander angeordnete Klemmbacken 9, 10 des Instrumentes 1 eingeschoben, und diese Klemmbacken 9, 10 werden einander angenähert, dabei verformen sie den Brückenabschnitt 7 der Ligaturklammern 4 und bewegen die Schenkel 5, 6 im Wesentlichen parallel zu sich selbst gegeneinander.

Die beiden Klemmbacken 9, 10 befinden sich an einem Bauteil, das im Inneren des Schaftes 2 in dem im Querschnitt halbkreisförmigen Aufnahmeraum innerhalb des Schaftes 2 angeordnet ist, der neben dem Magazin 3 liegt und von diesem nicht ausgefüllt ist.

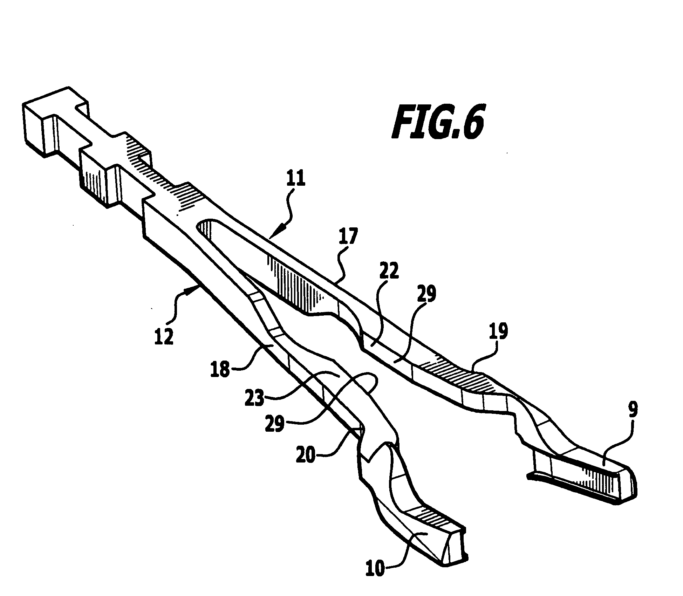

Dieses Bauteil (Figur 6) weist zwei nebeneinander liegende Arme 11, 12 auf, die am proximalen Ende des Bauteils miteinander verbunden sind und die jeweils an ihrem distalen Ende einen der beiden Klemmbacken 9 beziehungsweise 10 tragen. Das Bauteil ist einstückig ausgebildet und besteht aus einem elastisch verformbaren Material, insbesondere aus einem elastisch verformbaren Metall. Die Arme 11, 12 sind dadurch elastisch verbiegbar, sie können also aus der leicht aufgespreizten unverformten Stellung (Figur 6) elastisch gegeneinander verschwenkt werden, so dass sich dabei die beiden Klemmbacken 9, 10 einander annähern.

Die Arme 11, 12 sind in eine Kammer 13 eines Schiebers 14 eingeschoben, der die Arme über den größten Teil ihrer Länge umgibt. Der Schieber 14 hat einen halbkreisförmigen Querschnitt und füllt den Aufnahmeraum des Schaftes 2 neben dem Magazin 3 vollständig aus, so dass der Schieber 14 an der Innenwand des Schaftes 2 anliegt und in diesem längsverschieblich geführt ist. Die Kammer 13 hat zwei parallele, sich über den größten Teil der Länge des Schiebers erstreckende und im Abstand zueinander angeordnete Seitenwände 15, 16, und an diese Seitenwände 15, 16 legen sich die Außenseiten 17, 18 der Arme 11, 12 an, wenn die Arme in die Kammer 13 eingeschoben sind. Die Außenseiten verlaufen dann im Wesentlichen parallel zueinander, in dieser Stellung befinden sich die Arme 11, 12 in einer Anfangsstellung.

Die Außenseiten 17, 18 der Arme 11, 12 erweitern sich bei dem Austritt der Arme 11, 12 am distalen Ende des Schiebers 14 und bilden dort schräg nach außen verlaufende Anlageflächen 19, 20 aus, an denen der Schieber 14 mit seinem distalen Ende 21 anliegt. Unmittelbar an diese Anlageflächen 19, 20 schließen sich die beiden Klemmbacken 9, 10 an.

Durch die Anlage des distalen Endes 21 des Schiebers 14 an den Anlageflächen 19, 20 drückt der Schieber beim Vorschieben des Schiebers relativ zu den Armen in distaler Richtung die beiden Arme 11, 12 gegeneinander, das heißt diese werden durch das Entlanggleiten des distalen Endes 21 an den Anlageflächen 19, 20 gegeneinander verschwenkt, dabei werden die Arme 11, 12 elastisch nach innen gebogen. Dies führt zu einer Annäherung der Klemmbacken 9, 10, das heißt zu einer Bewegung der Klemmbacken von der Offenstellung in die Schließstellung.

Die Verschiebung zwischen dem Schieber und den Armen erfolgt durch Kopplung des Schiebers oder der Arme mit einem der Übertragungsmittel, die den Schaft 2 durchsetzen, es ist möglich, den Schieber im Schaft längsverschieblich zu lagern und gegenüber den Armen, die mit dem Schaft unverschieblich verbunden sind, zu verschieben, es ist aber umgekehrt auch möglich, den Schieber relativ zum Schaft festzuhalten und dafür die Arme in Längsrichtung zu verschieben. Wesentlich ist lediglich, dass sich die Arme und der Schieber in Längsrichtung relativ zueinander verschieben, so dass dadurch ein Entlanggleiten des distalen Endes 21 des Schiebers 14 an den Anlageflächen 19, 20 erfolgt.

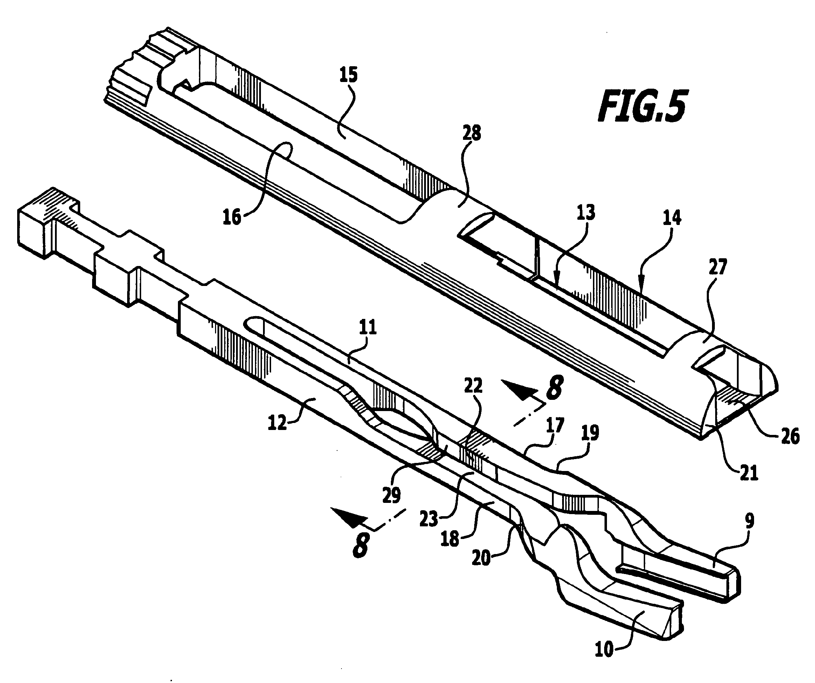

Im Inneren des Kammer 13 sind die Arme 11, 12 so angeordnet, dass sie sich zumindest bereichsweise überlappen, wie dies besonders deutlich aus der Darstellung der Figur 5 hervorgeht. Bei dem dort dargestellten Ausführungsbeispiel haben die Arme ausgehend vom proximalen Ende zunächst eine gleich bleibende Höhe, etwa in der Mitte der Arme reduziert sich bei beiden Armen die Höhe etwa auf die Hälfte, und zwar derart, dass sich ein Arm in einer unteren Ebene befindet und der andere Arm in einer oberen Ebene, die so weit gegeneinander versetzt sind, dass die Arme in dem Bereich reduzierter Höhe nicht mehr nebeneinander liegen sondern übereinander. Dadurch ist es möglich, die Arme gegeneinander zu verschwenken, ohne dass sie sich gegenseitig bei dieser Verschwenkbewegung in ihrer jeweiligen Bewegung stören. Die Überlappung kann bereits, wie in Figur 5 dargestellt, in der Ruhestellung erfolgen, also vor dem Verschwenken der Arme in die Schließstellung der Klemmbacken, beim Verschwenken der Arme in die Schließstellung der Klemmbacken wird die Überlappung noch verstärkt, bis sich die überlappenden Armbereiche 22, 23 gegebenenfalls vollständig überdecken. Durch die Überlappung der beiden Arme steht für jeden Arm bei der Verschwenkbewegung die vollständige Breite der Kammer 13 zur Verfügung, so dass ein relativ großer Winkelbereich bei der Schließbewegung der Arme überstrichen werden kann.

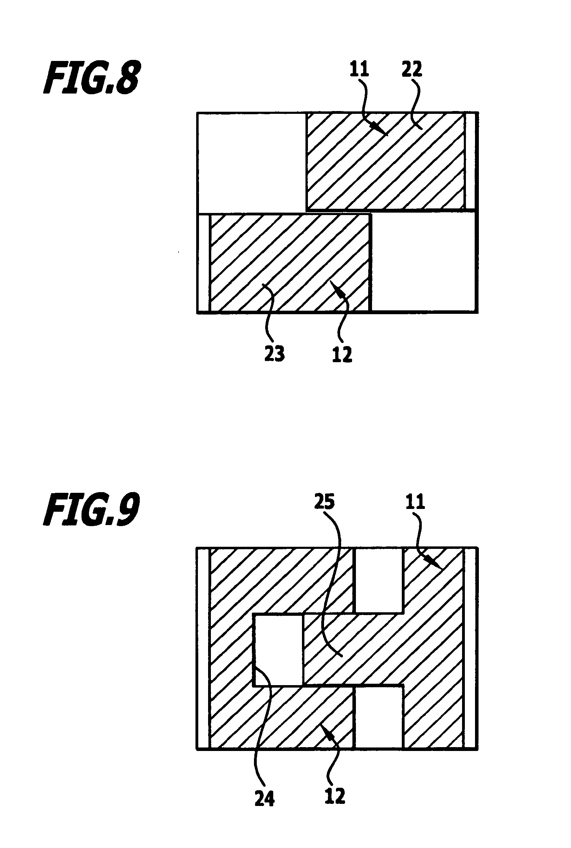

Bei dem in Figur 5 dargestellten Ausführungsbeispiel sind die Arme im überlappenden Bereich 22, 23 breiter ausgebildet als in dem proximal sich anschließenden Bereich, in dem die Arme die volle Höhe aufweisen. Diese Verbreiterung ist möglich, weil durch die Anordnung der Arme in unterschiedlichen Ebenen in Verschwenkrichtung Platz gewonnen worden ist, und diese Verbreiterung führt dazu, dass das Biegemoment der Arme im überlappenden Bereich 22 trotz der reduzierten Höhe beibehalten oder sogar vergrößert werden kann. Wie aus der Darstellung der Figur 8 ersichtlich wird, kann der Querschnitt der überlappenden Bereiche 22, 23 beispielsweise ein rechteckiger Querschnitt sein, bei dem die Breite größer ist als die Höhe. Die Breite der Arme, also die Erstreckung in Verschwenkrichtung, beeinflusst das Biegemoment der Arme in der jeweiligen Verschwenkebene quadratisch, während die Höhe dies nur linear beeinflusst. Dadurch kann die Reduzierung des Biegemomentes durch Reduzierung der Höhe ohne weiteres durch eine relativ geringfügige Verbreiterung der Arme in diesem Bereich kompensiert oder sogar überkompensiert werden.

Die Überlappung der beiden Arme ist bei dem in Figur 5 dargestellten Ausführungsbeispiel nur über einen Teilbereich der Länge der Arme vorgesehen, der sich im Wesentlichen an das distale Ende der Arme im proximaler Richtung anschließt, es wäre aber auch möglich, die Arme sich über deren gesamte Länge überlappen zu lassen.

Wesentlich ist, dass sich die Arme durch ihre Versetzung in unterschiedliche Ebenen bei der Verschwenkbewegung nicht gegenseitig stören, so dass für beide Arme ein größerer Verschwenkbereich zur Verfügung steht.

Bei der beschriebenen Ausführung befinden sich aufgrund der Versetzung der beiden Arme in unterschiedliche Ebenen auch die Anlageflächen 19, 20 in unterschiedlichen Ebenen, so dass auch die Anlage dieser Anlageflächen 19, 20 an dem Schieber 14 in unterschiedlichen Ebenen erfolgt.

Bei der Ausführung der Figur 5 befinden sich die überlappenden Bereiche in unterschiedlichen Ebenen, das heißt es gibt einen oberen überlappenden Bereich des einen Armes und einen unteren überlappenden Bereich des anderen Armes, wie es auch in Figur 8 dargestellt ist.

Es wäre auch möglich, andere Querschnitte der Arme zu verwenden, beispielsweise könnte, wie in Figur 9 dargestellt, ein Arm eine nutförmige Ausnehmung 24 aufweisen, in die der zweite Arm mit einem entsprechenden leistenförmigen Vorsprung 25 eingreift. Auch dies führt zu einer Überlappung der beiden Arme und zu einer Erhöhung der Biegemomente, trotzdem behindern sich die beiden Arme in dem Überlappungsbereich nicht gegenseitig, so dass eine größere Verschwenkung möglich ist als bei Armen, die ohne gegenseitigen Eingriff nebeneinander angeordnet sind und entsprechende Biegemomente aufweisen. Das Optimum der Verschwenkbarkeit wird natürlich durch eine Ausgestaltung erreicht, bei der die Arme vollständig in unterschiedlichen Ebenen angeordnet sind und somit die gesamte Breite der Kammer für ihre Verschwenkbewegung zur Verfügung haben.

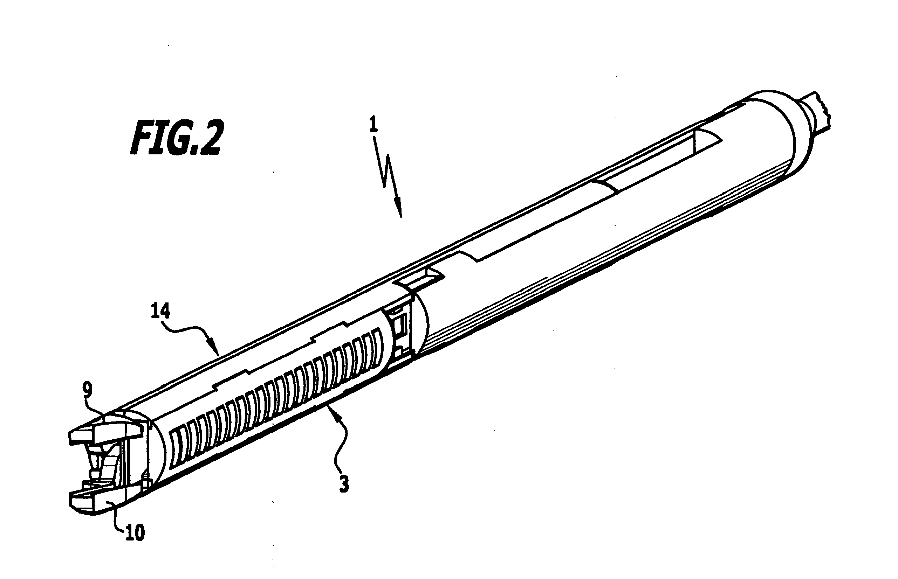

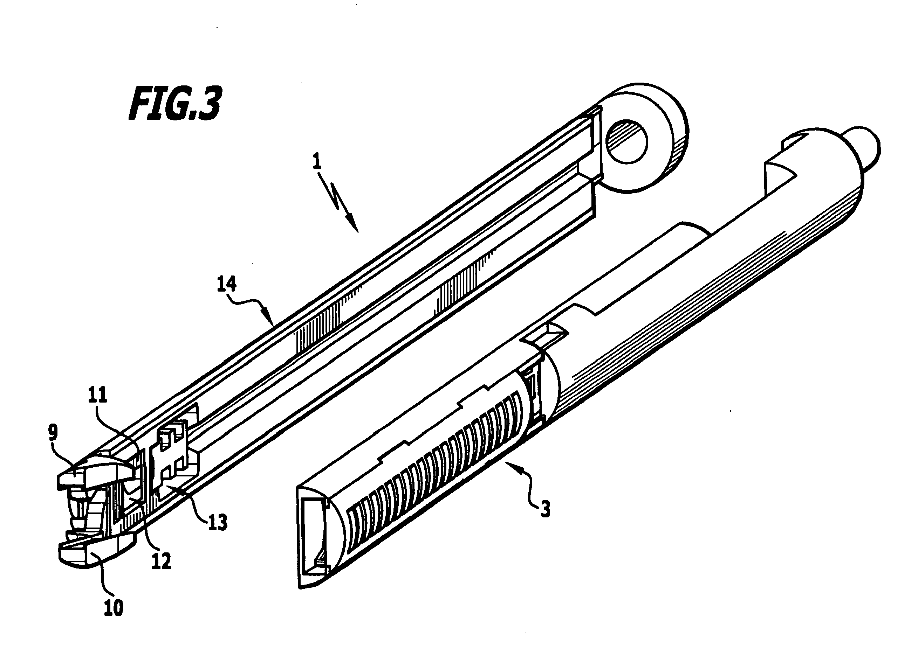

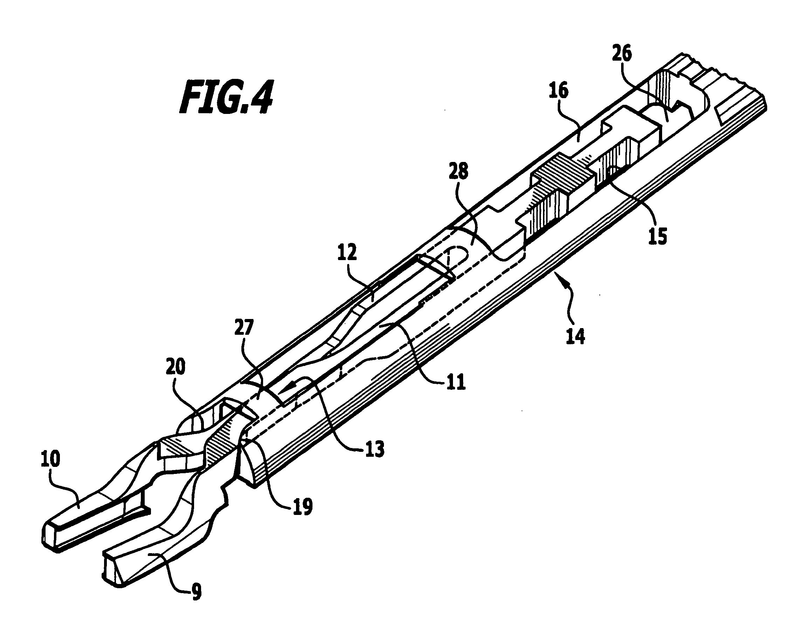

Der die Arme 11, 12 aufnehmende Schieber 14 ist bei dem Ausführungsbeispiel der Figuren 1 bis 5 so ausgebildet, dass der Schieber die Arme allseits umgibt. Dazu weist die Kammer 13 an ihrer Unterseite einen die beiden Seitenwände 15, 16 verbindenden ebenen Boden und an der Oberseite die Seitenwände 15, 16 verbindende Stege 27, 28 auf, das heißt zumindest im Bereich der Stege 27, 28 sind die Arme von dem Schieber allseits umschlossen, so dass sich dadurch eine relativ große Stabilität der aus dem Schieber und den Armen bestehenden Baueinheit ergibt.

Allerdings ist bei dieser Ausgestaltung ein Teil des innerhalb des Schaftes 2 zur Verfügung stehenden Querschnittes durch die Seitenwände 15, 16 ausgefüllt, und dieser Teil des Querschnittes steht für die Verschwenkbewegung der Arme 11, 12 daher nicht zur Verfügung.

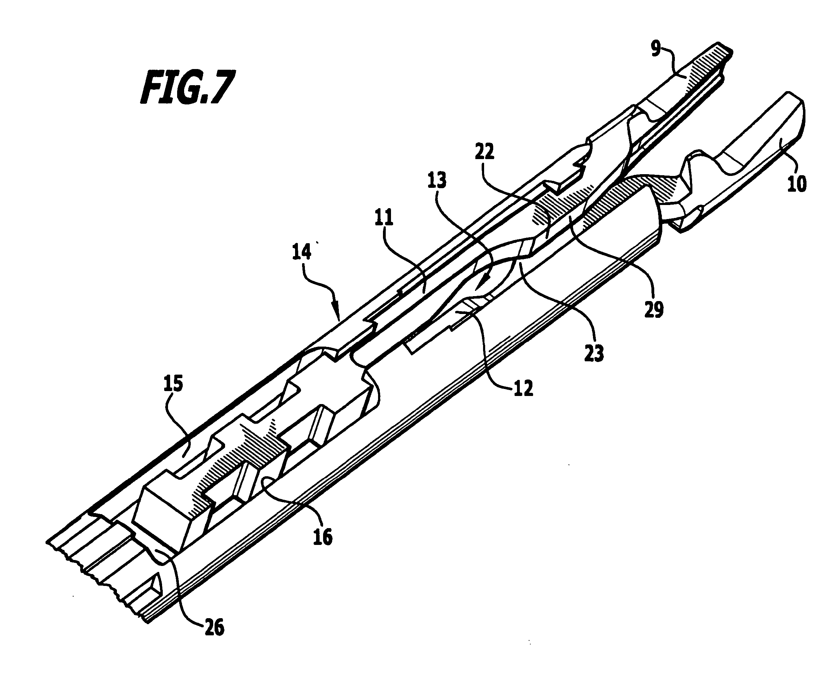

Um den gesamten Querschnitt des Schaftes ausnützen zu können, ist bei dem Ausführungsbeispiel der Figur 7 der Schieber nicht mehr so ausgebildet, dass er die Kammer 13 allseits umgibt, sondern bei diesem Ausführungsbeispiel sind die Stege weggelassen und die Höhe der Seitenwände 15, 16 ist so begrenzt, dass den Innenflächen 29 der Arme 11, 12 kein Material des Schiebers 14 gegenüberliegt, sich also die Arme 11, 12 bis an den Rand der durch den Schaft 2 vorgegebenen halbkreisförmigen Kontur erstrecken können. Dadurch wird der Schwenkbereich der Arme vergrößert, allerdings führt dies auch zu

## Patentansprüche

1. Chirurgisches Instrument (1) zum Anlegen von C-förmigen, zwei über einen Brückenabschnitt miteinander verbundene Schenkel (5, 6) aufweisenden Ligaturklammern (4) mit zwei in einer Offenstellung eine offene Ligaturklammer (4) zwischen sich aufnehmenden und durch gegenseitige Annäherung in eine Schließstellung bewegbaren und dabei die Ligaturklammer (4) durch Annäherung ihrer Schenkel (5, 6) schließenden Klemmbacken (9, 10), mit zwei elastisch verformbaren Armen (11, 12), die an ihrem distalen Ende je einen Klemmbacken (9, 10) tragen, und mit einem in Längsrichtung der Arme (11, 12) relativ zu diesen verschiebbaren Schieber (14), der an Anlageflächen der Arme (11, 12) anliegt und bei einer Verschiebung des Schiebers (14) relativ zu den Armen (11, 12) durch diese Anlage an den Anlageflächen die Arme (11, 12) in einer Verschwenkebene elastisch aus einer Anfangsstellung in eine Endstellung gegen einander schwenkt, wobei die beiden Arme (11, 12) zumindest über einen Teil ihrer Länge und zumindest über einen Teil ihrer Breite in parallel zu der Verschwenkebene verlaufenden und gegen einander versetzten Ebenen angeordnet sind und sich zumindest teilweise überlappen, **dadurch gekennzeichnet, dass** im Überlappungsbereich den innen liegenden Seitenflächen (29) der Arme (11, 12) bei deren Verschwenkung in die Endstellung kein Teil des Schiebers (14) gegenüber liegt.

2. Instrument nach Anspruch 1, **dadurch gekennzeichnet, dass** einer der Arme (11) im Überlappungsbereich vollständig in einer oberen Ebene angeordnet ist und der andere der Arme (12) in einer unteren Ebene.

3. Instrument nach Anspruch 1, **dadurch gekennzeichnet, dass** einer der Arme (11) im Überlappungsbereich eine zum anderen Arm (12) hin offene Ausnehmung (24) aufweist, in die der andere Arm (12) zumindest teilweise eintaucht.

4. Instrument nach einem der voranstehenden Ansprüche, **dadurch gekennzeichnet, dass** sich die Arme (11, 12) bereits in ihrer Anfangsstellung, in der die Klemmbacken (9, 10) in der Offenstellung stehen, teilweise überlappen.

5. Instrument nach einem der voranstehenden Ansprüche, **dadurch gekennzeichnet, dass** sich die Arme (11, 12) in ihrer Endstellung, in der die Klemmbacken (9, 10) in der Schließstellung stehen, zumindest bereichsweise über ihre gesamte Breite überlappen.

6. Instrument nach einem der voranstehenden Ansprüche, **dadurch gekennzeichnet, dass** die Breite der Arme (11, 12) im Überlappungsbereich größer ist als deren Höhe.

7. Instrument nach einem der voranstehenden Ansprüche, **dadurch gekennzeichnet, dass** die außen liegenden Seitenflächen (17, 18) der Arme (11, 12) in der Anfangsstellung über die gesamte Länge der Arme (11, 12) mit Ausnahme des distalen Endbereiches innerhalb einer Kontur mit parallel zueinander verlaufender Begrenzung angeordnet sind.

8. Instrument nach Anspruch 7, **dadurch gekennzeichnet, dass** die Begrenzung durch die Seitenwände (15, 16) einer die Arme (11, 12) aufnehmenden Kammer (13) des Schiebers (14) gebildet wird.

9. Instrument nach einem der voranstehenden Ansprüche, **dadurch gekennzeichnet, dass** der Schieber (14) an seinem distalen Ende an nach außen abstehenden Anlageflächen (19, 20) der Arme (11, 12) anliegt, die sich unmittelbar proximal an die Klemmbacken (9, 10) anschließen.

10. Instrument nach Anspruch 9, **dadurch gekennzeichnet, dass** die Anlageflächen (19, 20) der Arme (11, 12) in unterschiedlichen Ebenen liegen.

11. Instrument nach einem der Ansprüche 9 oder 10, **dadurch gekennzeichnet, dass** an einem Arm die Anlageflächen (19, 20) in der Ebene des entsprechenden Armes liegen.

12. Instrument nach einem der voranstehenden Ansprüche, **dadurch gekennzeichnet, dass** die Arme (11, 12) zumindest an einem Teil ihrer Länge von dem Schieber (14) allseits umgeben werden.

13. Instrument nach einem der voranstehenden Ansprüche, **dadurch gekennzeichnet, dass** sich die Arme (11, 12) im unverformten Zustand über ihre gesamte Länge nicht überlappen.

14. Instrument nach einem der voranstehenden Ansprüche, **dadurch gekennzeichnet, dass** sich die Arme (11, 12) zwischen ihrem proximalen Ende und ihrem distalen Ende überkreuzen.

15. Instrument nach einem der voranstehenden Ansprüche, **dadurch gekennzeichnet, dass** Arme (11, 12) und der Schieber (14) gemeinsam innerhalb eines rohrförmigen Schaftes (2) mit einer kreisförmigen Kontur angeordnet sind, über die auch die Klemmbacken (9, 10) in ihrer Offenstellung nicht hervorstehen. einer Herabsetzung der Festigkeit des Schiebers 14 im distalen Bereich. Dies kann jedoch dadurch kompensiert werden, dass sich der Schieber 14 mit seiner Außenfläche an der Innenfläche des Schaftes 2 abstützt und dadurch eine zusätzliche Führung erfährt.

## Claims

1. A surgical instrument (1) for the placement of C-shaped ligature clips (4) having two legs (5, 6) connected to one another by means of a bridge section with two clamping jaws (9, 10), which in an open position receive an open ligature clip (4) between them and are movable into a closed position by approaching one another and in so doing close the ligature clip (4) by its legs (5, 6) approaching one another, with two elastically deformable arms (11, 12), each bearing a clamping jaw (9, 10) at its distal end, and with a slide (14), which is displaceable relative to the arms (11, 12) in the longitudinal direction thereof, and which abuts against abutment surfaces of the arms (11, 12) and as a result of this abutment against the abutment surfaces elastically pivots the arms (11, 12) relative to one another in a pivoting plane out of a starting position into an end position upon displacement of the slide (14) relative to the arms (11, 12), wherein at least over a part of their length and at least over a part of their width the two arms (11, 12) are arranged in planes running parallel to the pivoting plane and displaced relative to one another and overlap one another at least partially, **characterised in that** in the overlap region no part of the slide (14) lies opposite the side faces (29) of the arms (11, 12) located on the inside and strikes these when they pivot into the end position.

2. An instrument according to claim 1, **characterised in that** in the overlap region one of the arms (11) is arranged completely in an upper plane and the other arm (12) in a lower plane.

3. An instrument according to claim 1, **characterised in that** in the overlap region one of the arms (11) has a recess (24) open towards the other arm (12), into which recess the other arm (12) projects as least partially.

4. An instrument according to one of the preceding claims, **characterised in that** the arms (11, 12) already overlap partially in their starting position, in which the clamping jaws (9, 10) are in the open position.

5. An instrument according to one of the preceding claims, **characterised in that** in their end position, in which the clamping jaws (9, 10) are in the closed position, the arms (11, 12) overlap at least in some areas over their entire width.

6. An instrument according to one of the preceding claims, **characterised in that** the width of the arms (11, 12) in the overlap region is greater than their height.

7. An instrument according to one of the preceding claims, **characterised in that** in the starting position the side faces (17, 18) of the arms (11, 12) located on the outside are arranged within a contour with bounding means running parallel to one another over the entire length of the arms (11, 12) with the exception of the distal end region.

8. An instrument according to claim 7, **characterised in that** the bounding means is formed by the side walls (15, 16) of a chamber (13) of the slide (14) receiving the arms (11, 12).

9. An instrument according to one of the preceding claims, **characterised in that** at its distal end the slide (14) abuts against outwardly projecting abutment surfaces (19, 20) of the arms (11, 12), which directly adjoin the clamping jaws (9, 10) in proximal direction.

10. An instrument according to claim 9, **characterised in that** the abutment surfaces (19, 20) of the arms (11, 12) lie in different planes.

11. An instrument according to one of claims 9 or 10, **characterised in that** on one arm the abutment surfaces (19, 20) lie in the plane of the corresponding arm.

12. An instrument according to one of the preceding claims, **characterised in that** the arms (11, 12) are surrounded on all sides by the slide (14) at least on a part of their length.

13. An instrument according to one of the preceding claims, **characterised in that** in the non-deformed state the arms (11, 12) do not overlap over their entire length.

14. An instrument according to one of the preceding claims, **characterised in that** the arms (11, 12) intersect between their proximal end and their distal end.

15. An instrument according to one of the preceding claims, **characterised in that** the arms (11, 12) and the slide (14) are arranged together inside a tubular barrel (2) with a circular contour, beyond which the clamping jaws (9, 10) likewise do not project in their open position.

## Revendications

1. Instrument chirurgical (1) pour la mise en place de pinces de ligature (4) en forme de C avec deux branches (5, 6) reliées l'une à l'autre par une partie formant pont, comportant deux mâchoires de serrage (9, 10) recevant, dans une position ouverte, entre elles une pince de ligature (4) ouverte et pouvant être amenées en position de fermeture en les rapprochant l'une de l'autre pour fermer la pince de ligature (4) sous l'effet du rapprochement de ses branches (5, 6), comportant deux bras (11, 12) élastiquement déformables qui portent chacun au niveau de leur extrémité distale une mâchoire de serrage (9, 10), et comportant une coulisse (14), qui est mobile dans le sens longitudinal des bras (11, 12) par rapport à ceux-ci et qui est en appui contre des surfaces d'appui des bras (11, 12) et, lors d'un mouvement de translation de la coulisse (14) par rapport aux bras (11, 12), fait pivoter, sous l'effet de l'appui contre les surfaces d'appui des bras (11, 12), lesdits bras élastiquement l'un contre l'autre dans un plan de pivotement hors d'une position initiale vers une position de fin de course, lesdits deux bras (11, 12) étant disposés au moins sur une partie de leur longueur et au moins sur une partie de leur largeur dans des plans orientés parallèlement au plan de pivotement et décalés entre eux, et se chevauchant au moins en partie, **caractérisé en ce que**, dans la zone de chevauchement, aucune partie de la coulisse (14) n'est située en face des surfaces latérales (29) intérieures des bras (11, 12) et n'est en contact avec elles lors de leur pivotement dans la position de fin de course.

2. Instrument selon la revendication 1, **caractérisé en ce que**, dans la zone de chevauchement, un des bras (11) est agencé intégralement dans un plan supérieur et l'autre bras (12) est agencé intégralement dans un plan inférieur.

3. Instrument selon la revendication 1, **caractérisé en ce que**, dans la zone de chevauchement, un des bras (11) comporte un évidement (24) ouvert vers l'autre bras (12), dans lequel l'autre bras (12) pénètre au moins partiellement.

4. Instrument selon l'une quelconque des revendications précédentes, **caractérisé en ce que** les bras (11, 12) se chevauchent partiellement déjà dans leur position initiale, dans laquelle les mâchoires de serrage (9, 10) sont en position ouverte.

5. Instrument selon l'une quelconque des revendications précédentes, **caractérisé en ce que** les bras (11, 12) se chevauchent au moins par zones sur toute leur largeur dans leur position de fin de course, dans laquelle les mâchoires de serrage (9, 10) sont en position fermée.

6. Instrument selon l'une quelconque des revendications précédentes, **caractérisé en ce que** la largeur des bras (11, 12) dans la zone de chevauchement est supérieure à leur hauteur.

7. Instrument selon l'une quelconque des revendications précédentes, **caractérisé en ce que** les surfaces latérales (17, 18) extérieures des bras (11, 12) dans la position initiale sont disposées sur toute la longueur des bras (11, 12), à l'exception de la zone d'extrémité distale, à l'intérieur d'un contour avec des délimitations parallèles entre elles.

8. Instrument selon la revendication 7, **caractérisé en ce que** les délimitations sont formées par les parois latérales (15, 16) d'une chambre (13) de la coulisse (14), recevant les bras (11, 12).

9. Instrument selon l'une quelconque des revendications précédentes, **caractérisé en ce que** la coulisse (14), au niveau de son extrémité distale, est en appui contre des surfaces d'appui (19, 20) saillantes vers l'extérieur sur les bras (11, 12) et prolongeant directement en proximal les mâchoires de serrage (9, 10).

10. Instrument selon la revendication 9, **caractérisé en ce que** les surfaces d'appui (19, 20) des bras (11, 12) sont situées dans des plans différents.

11. Instrument selon la revendication 9 ou 10, **caractérisé en ce que**, sur un bras, les surfaces d'appui (19, 20) sont situées dans le plan du bras correspondant.

12. Instrument selon l'une quelconque des revendications précédentes, **caractérisé en ce que** les bras (11, 12) sont entourés de toutes parts au moins sur une partie de leur longueur par la coulisse (14).

13. Instrument selon l'une quelconque des revendications précédentes, **caractérisé en ce que** les bras (11, 12), à l'état non déformé, ne se chevauchent pas sur toute leur longueur.

14. Instrument selon l'une quelconque des revendications précédentes, **caractérisé en ce que** les bras (11, 12) se croisent entre leur extrémité proximale et leur extrémité distale.

15. Instrument selon l'une quelconque des revendications précédentes, **caractérisé en ce que** les bras (11, 12) et la coulisse (14) sont agencés conjointement à l'intérieur d'une tige (2) tubulaire avec un contour circulaire, au-delà duquel les mâchoires de serrage (9, 10) ne s'avancent pas non plus dans leur position ouverte.
